# EUROPÄISCHE PATENTSCHRIFT

(11) **EP 2 025 362 B1**
(45) Veröffentlichungstag und Bekanntmachung des Hinweises auf die Patenterteilung: **14.12.2011**
(21) Anmeldenummer: 08160210.4
(22) Anmeldetag: 11.07.2008
(51) Int. Cl.: A61N 1/36

(54) **Stimulationssystem zur Behandlung von Dysphagien**
Stimulation system for treating dysphagia
Système de stimulation destiné au traitement de dysphagies

(30) Priorität: 16.08.2007 DE 102007038816
(43) Veröffentlichungstag der Anmeldung: 18.02.2009
(73) Patentinhaber: Biotronik CRM Patent AG, 6341 Baar (CH)
(72) Erfinder: Philipp, Jens, Dr., 10555 Berlin (DE); Kautz, Dirk, Dr., 15831 Mahlow (DE); Feußner, Hubertus, Prof. Dr., 81671 München (DE)
(74) Vertreter: Lindner-Vogt, Karin L.

(56) Entgegenhaltungen:
- WO-A-2007/005582
- US-A- 5 891 185
- US-A1- 2003 078 521
- BURNETT THERESA A ET AL: "Self-triggered functional electrical stimulation during swallowing" JOURNAL OF NEUROPHYSIOLOGY, AMERICAN PHYSIOLOGICAL SOCIETY, BETHESDA, MD, US, Bd. 94, Nr. 6, 17. August 2005 (2005-08-17), Seiten 4011-4018, XP007903287 ISSN: 0022-3077

## Beschreibung

Die Erfindung betrifft ein implantierbares medizinisches System, insbesondere ein System zum Auslösen des Schluckaktes bei Schluckstörungen, der sogenannten zervikalen Dysphagie.

Schluckstörungen entstehen zum Beispiel dann, wenn nach Beginn des willentlich initiierten Schluckvorganges der Speisebrei nicht korrekt in die Speiseröhre geleitet wird, weil der Schließmuskel am Kreuzungspunkt zwischen Luftröhre und Speiseröhre (der Ösophagussphinkter) nur unzureichend oder gar nicht aktiviert wird. Diese Schluckstörung kann beispielsweise durch Schädigung bestimmter Gehirnareale oder der verbindenden Nerven verursacht werden und ist häufig auch eine Folge von Schlaganfällen.

Dabei gehören Schluckstörungen zu denjenigen körperlichen Beeinträchtigungen, die die Lebensqualität der Patienten besonders nachhaltig reduzieren. Darüber hinaus stellt die zervikale Dysphagie eine konkrete gesundheitliche Bedrohung insbesondere durch Folgekomplikationen wie Unterernährung, Dehydrierung, Atemprobleme, Abhängigkeit von Sondenernährung und/oder einer Trachealkanülen, Tod, sowie hohe Kosten für das Gesundheitssystem dar.

Eine weitere Gefahr besteht darin, dass unzerkaute Nahrung und noch zu große Bissen "im Halse stecken bleiben" und möglicherweise sogar zu einer Obstruktion der Atemwege führen. Dies kann dadurch zustande kommen, dass der Speisebrei bei nicht oder unzureichend aktiviertem Ösophagussphinkter anstatt in die Speiseröhre zu gelangen in die Luftröhre rutscht. Atemstillstand und Herztod sind nicht selten die Folge.

Verdeutlicht wird dies insbesondere auch durch die Tatsache, dass Dysphagien eine der häufigsten Todesursachen von Patienten innerhalb des ersten Jahres nach einem Schlaganfall sind.

Durch die zunehmende Lebenserwartung und die damit verbundenen neurologischen (Schlaganfall) sowie onkologischen Erkrankungen (Krebs) im Kopf-Hals-Gebiet steigt die Zahl der Patienten, die unter Schluckstörungen (Dysphagien) leiden, kontinuierlich an.

Beim Schlaganfall (Inzidenz 24/100.000, Prävalenz 1.200/100.000 in den westlichen Industrieländern) leiden über 50% der Betroffenen in der Akutphase an klinisch relevanter Dysphagie. Von diesen 50% versterben etwa die Hälfte oder erholen sich ausreichend, sodass etwa 25% der Schlaganfall-Patienten eine chronische Dysphagie aufweisen.

Eine zweite, ständig wachsende Gruppe umfasst den Bereich der alten Menschen. So treten Dysphagien unter älteren Heimbewohnern in 30 - 60% auf. Bei fortgeschrittener Demenz liegt fast immer eine Dysphagie vor. Zahlreiche andere und häufig neurologische Erkrankungen gehen mit Dysphagien einher. So leiden Patienten mit Morbus Parkinson (Prävalenz 300 - 1.000/100.000) zu ca. 40 - 50% an Dysphagien, Patienten mit Multipler Sklerose (Prävalenz 100/100.000) zu ca. 30 - 40%.

Zur Behandlung der zervikalen Dysphagie sind aus dem Stand der Technik körperexterne Systeme, wie z. B. das VitalStim® System bekannt, welches zur neuromuskulären Elektrostimulation dient und während des Schluckvorgangs von außen an den Bereich der vorderen Halsregion angelegt wird um eine Kontraktion der Schluckmuskulatur zu bewirken.

Diese Stimulation der Schluckmuskulatur zielt aber lediglich auf eine Aktivierung einzelner oder mehrerer Muskeln, nicht aber auf die Initiierung des gesamten Schluckablaufs ab. Ergebnisse von Studien zu solchen Systemen waren bisher enttäuschend.

Untersuchungen zur elektrischen Stimulation u. a. im Bereich des Rachens, der Gaumenbögen und in der Halsregion mit unterschiedlicher Frequenz und Intensität wurden und werden durchgeführt bzw. hinsichtlich ihrer Wirksamkeit, d.h. der positiven Beeinflussung relevanter Schluckparameter, erprobt, haben jedoch bislang noch nicht zu einem System geführt, welches einem Patienten ein nahezu normales und natürliches Schluckverhalten ermöglicht.
Des Weiteren beschreibt die US5.891.185 ein externes Stimulationsgerät zum Auslösen des Schluckreflexes.

In dem Artikel "Self-Ttriggered Functional Electrical Stimulation During Swalluwing" im J. Neurophysiol 94:4011-4018, 2005 von T.A. Burnett et al. wird ein Gerät zur Schluckstimulation mit zumindest teilweise implantierbaren Elektroden beschrieben.

Die US20030078521 beschreibt ein Gerät, mit dem die Zungenkraft gemessen und die Zunge trainiert werden kann.

Es ist demnach die Aufgabe dieser Erfindung, ein System bereit zu stellen, das die oben genannten Nachteile löst.

Die genannte Aufgabe wird erfindungsgemäß durch ein voll implantierbares Stimulationssystem gelöst, welches ausgelöst durch eine willkürliche Muskelbewegung, einen willentlich hervorgerufenen Gehirnstrom oder einen willentlich erzeugten Nervenimpuls eines Patienten während der Initialphase eines Schluckvorgangs eine Stimulation des reflektorischen Ablaufs des Schluckvorgangs bewirkt.

Unter dem Begriff "willentlich hervorgerufener Gehirnstrom" sind solche messbaren elektrischen Aktivitäten des Gehirns zu verstehen, die "trainierbar" sind und von einem Patienten bewusst zum Initialisieren eines Schluckvorgangs ausgelöst werden können. Unter dem Begriff "willentlich erzeugter Nervenimpuls" sind solche messbaren Nervenimpulse zu verstehen, welche ebenfalls "trainierbar" sind und durch einen Patienten bewusst ausgelöst werden können. Analog bezieht sich der Begriff "willkürliche Muskelbewegung" auf solche Muskelbewegungen, welche willentlich gesteuert werden können.

Dabei umfasst das erfindungsgemäße Stimulationssystem ein implantierbares Stimulationsgerät, wenigstens eine implantierbare Stimulationselektrode und wenigstens einen Initialisierungs-Sensor.

Der Initialisierungs-Sensor ist dabei dazu ausgebildet, im Behandlungsfall eine willentlich gesteuerte Muskelbewegung, einen willentlich hervorgerufenen Gehirnstrom oder einen willentlich erzeugten Nervenimpuls eines Patienten während der Initialphase eines Schluckvorgangs zu detektieren, kontinuierlich, zeitlich getaktet oder in Reaktion hierauf ein Sensor-Signal zu erzeugen und dieses an das Stimulationsgerät weiterzugeben. Das Stimulationsgerät ist dabei seinerseits dazu ausgebildet, bei solch einem Sensor-Signal des Initialisierungs-Sensors, das eine willentliche Initiierung eines Schluckvorgangs repräsentiert, die Abgabe eines Stimulationsimpulses über die Stimulationselektrode auszulösen.

Ein Signal, welches die Abgabe eines Stimulationsimpulses auslöst, wird im Kontext dieser Erfindung auch als auslösendes Sensor-Signal bzw. Triggersignal bezeichnet. Für eine solche Ausführungsform der Erfindung, in der der Initialisierungs-Sensor ausschließlich im Falle einer tatsächlich vorliegenden Muskelbewegung, eines tatsächlich willentlich hervorgerufenen Gehirnstroms oder eines tatsächlich willentlich erzeugten Nervenimpulses während der Initialphase eines Schluckvorgangs ein Sensor-Signal erzeugt und dieses an das Stimulationsgerät abgibt, handelt es sich bei dem Sensor-Signal dementsprechend um ein Triggersignal. Für eine solche Ausführungsform, bei der der Initialisierungs-Sensor allerdings kontinuierlich ein Sensor-Signal an das Stimulationsgerät abgibt, müssen aus diesem Signal - wie weiter unten noch ausführlich erläutert - im Zuge einer Signalaufbereitung erst noch die auslösenden Sensor-Signale bzw. die Triggersignale "herausgefiltert" werden.

Implantiert im Körper eines Patienten wird mit Hilfe des erfindungsgemäßen Stimulationssystems durch eine willentlich gesteuerte Muskelbewegung, einen willentlich hervorgerufenen Gehirnstrom oder einen willentlich erzeugten Nervenimpuls zur Initiierung eines Schluckvorgangs der reflektorische Schluckvorgang des Patienten ausgelöst.

Dabei ist der Initialisierungs-Sensor - im Folgenden auch Sensor genannt - als Drucksensor und besonders bevorzugt Drucksensor für orientierte Druckmessung im Bereich von 30 bis 500 mmHg bei einer Auflösung von ca. delta 5 bis 10 mmHg und einer Frequenz von 2 bis 5 Hz. ausgeführt und besonders bevorzugt dazu ausgebildet, einen Druck der Zunge beziehungsweise eines Speisebolus gegen den Gaumen eines Patienten zu detektieren und daraufhin ein Sensor-Signal an das Stimulationsgerät auszugeben.

Prinzipiell ist aber jeder Sensor, welcher in der Lage ist, eine willentlich gesteuerte Muskelbewegung, einen willentlich hervorgerufenen Gehirnstrom oder einen willentlich erzeugten Nervenimpuls detektieren zu können, geeignet. Beispiele für solche Initialisierungs-Sensoren sind Augenbewegungsmesser, Elektrookulografie-Sensoren (EOG), Elektroenzephalografie-Sensoren (EEG) oder Drucksensoren, welche z. B. einen von extern ausgeübten Druck auf die Wange eines Patienten detektieren können. Je nach Ausgestaltung und gewünschtem Detektionsort sind solche Sensoren dazu ausgebildet, auf der Körperoberfläche oder implantiert im Körper des Patienten angeordnet zu werden. Sie verfügen dazu vorzugsweise über ein geeignetes Mittel zur Befestigung am oder im Körper eines Patienten.

Als Sensor-Signal für eine Stimulation des reflektorischen Schluckvorgangs dient besonders bevorzugt ein vorgegebener Anpressdruck der Zunge gegen den harten Gaumen. Ein Sensor, der einen solchen Druck detektiert, wird beispielsweise im oder auf dem Gaumen angebracht.

Zur Übertragung des Sensor-Signals an das Stimulationsgerät ist vorzugsweise der Sensor über eine Sensorelektrodenleitung elektrisch leitend mit dem Stimulationsgerät verbunden.

Gemäß einer weiteren Ausführungsvariante verfügt der Sensor jedoch über eine eigene Energieversorgung und ist dazu ausgebildet, ein Sensor-Signal drahtlos an das Stimulationsgerät zu übertragen.

Um zu verhindern, dass ein Sensor-Signal einen ungewollten Schluckvorgang auslöst und/oder um zu entscheiden, ob ein eingehendes Sensor-Signal auf eine willentlich gesteuerte Muskelbewegung, einen willentlich hervorgerufenen Gehirnstrom oder einen willentlich erzeugten Nervenimpuls während der Initialphase eines Schluckvorgangs zurückgeht, verfügt gemäß einer bevorzugten Ausführungsform der Sensor und/oder das Stimulationsgerät über eine Signalaufbereitung, um aus dem Sensor-Signal bzw. aus den Sensor-Signalen nur solche "herauszufiltern", die wirklich auf eine willentliche Initiierung eines Schluckvorgangs zurückgehen.

Die Signalaufbereitung ist dabei vorzugsweise dazu ausgebildet, mit Hilfe eines oder mehrerer Schwellenwerte und/oder mit Hilfe eines geforderten zeitlichen Verlaufsmusters des Sensor-Signals, eine solche Filterung vorzunehmen und dadurch eine ungewollte Stimulation des reflektorischen Ablaufs des Schluckvorgang zu verhindern.

Als Vergleichswert bzw. als Vergleichssignal dient bevorzugt ein charakteristischer und besonders bevorzugt ein patientenindividueller Druckverlauf am Gaumen eines Patienten. Gemäß weiteren Ausführungsformen dient als Vergleichswert oder Vergleichssignal ein patientenindividueller Druckverlauf im Bereich eines Muskels, eine patientenindividuelle Abfolge von Muskelbewegungen, ein patientenindividueller Gehirnstromverlauf oder eine patientenindividuelle Nervenimpulsabfolge während der Initialphase eines Schluckvorganges dazu, das Sensor-Signal aufzubereiten und auszuwerten.
Nach Eingehen des Sensor-Signals und ggf. nach einer Aufbereitung des Sensor-Signals gibt das Stimulationsgerät im implantierten Zustand bei Bedarf zeitgerecht einen oder mehrere Stimulationsimpulse über die Stimulationselektrode(n) an vorzugsweise einen Nervus laryngeus superior oder an beide Nervi laryngei superiori (die sich gegenüber liegen) ab, wodurch der Schluckablauf ausgelöst wird. Durch Stimulation dieser Nerven wird der gesamte oropharyngeale Schluckvorgang initiiert.

Um das Stimulationsgerät im Körper eines Patienten auch entfernt von dem Ort der Stimulationselektrode(n) implantieren zu können, verfügt das Stimulationsgerät vorzugsweise über einen Anschluss für eine Stimulationselektrodenleitung oder das Stimulationsgerät ist fest mit einer derartigen Elektrodenleitung verbunden. Über die Elektrodenleitung wird ein im Behandlungsfall generierter Stimulationsimpuls zu der Stimulationselektrode geleitet und über diese abgegeben.

Gemäß einer weiteren Ausführungsvariante verfügt das Stimulationssystem über wenigstens zwei Stimulationselektroden, welche über vorzugsweise jeweils eine eigene Stimulationselektrodenleitung mit dem Stimulationsgerät oder mit einer seiner Komponenten verbunden oder verbindbar sind.

Gemäß einer weiteren Ausführungsvariante verfügt die Stimulationselektrode über eine eigene Energieversorgung und eine drahtlose Datenübertragungseinheit mit vorzugsweise einem Sender und einem Empfänger und ist zum Empfang von Stimulationsimpulsen ausgebildet.

Bereits durch die elektrische Stimulation eines Nerven, des Nervus laryngeus superior wird im Anwendungsfall ein "Central Pattern Generator (CPG) for Swallowing" im Gehirn des Patienten aktiviert, der die Bewegung der einzelnen Schluckmuskeln geeignet koordiniert. Dabei reicht eine einseitige Stimulation des Nervus laryngeus superior zur Auslösung eines spatiotemporal geordnet ablaufenden Schluckvorgangs aus.

Weitere geeignete Stimulationsorte können andere Nerven oder Areale im Gehirn sein. Entsprechend dem gewünschten Stimulationsort ist das erfindungsgemäße Stimulationssystem mit wenigstens einer entsprechend angepassten Stimulationselektrode ausgestattet. Gemäß einer Ausführungsvariante wird die Elektrode durch eine Schraubenelektrode gebildet.

Das Stimulationsgerät selbst verfügt vorzugsweise über einen Impulsformer, eine Recheneinheit und einen Speicher und besonders bevorzugt auch über eine eigene Energieversorgung. Der Speicher des Stimulationsgerätes ist dazu ausgebildet, Parameter abzuspeichern, mit deren Hilfe die Recheneinheit eine vorzugsweise patientenindividuelle Stimulation bzw. Stimulationsabfolge errechnen und eine entsprechende Impulsgenerierung durch den Impulsformer bewirken kann. Die entsprechenden Parameter enthalten vorzugsweise Informationen, mit deren Hilfe eine Zeitverzögerung zwischen dem auszulösenden Sensor-Signal und der Abgabe des Stimulationssignals und/oder die Stimulationsfrequenz und/oder die Stimulationsart (Strom- oder Spannungsgesteuert) und/oder die Intensität der Stimulationsimpulse und/oder die Dauer eines Stimulationszyklus und/oder die Frequenz der Stimulationszyklen bestimmt und von dem Impulsformer generiert werden kann.

Ist das Stimulationsgerät wie oben beschrieben zur Aufbereitung des Sensor-Signals vorgesehen, so ist in diesem Falle bevorzugt die Recheneinheit dazu ausgebildet, diese Aufgabe zu übernehmen.

Gemäß einer besonders bevorzugten Ausführungsvariante ist das Stimulationsgerät darüber hinaus dazu ausgebildet, eine selbständige Anpassung der Stimulationsparameter durchführen zu können.

Gemäß einer weiteren Ausführungsvariante oder in Verbindung mit der Ausführungsvariante zur selbständigen Anpassung der Stimulationsparameter, verfügt das Stimulationssystem neben dem Initialisierungs-Sensor über wenigstens einen weiteren Sensor, welcher dazu ausgebildet ist, eine erfolgreiche Stimulation des reflektorischen Schluckablaufs zu detektieren.

Gemäß einer weiteren Ausführungsvariante verfügt das Stimulationsgerät über eine drahtlose Datenübertragungseinheit, welche dazu ausgebildet ist, zum Abspeichern in dem Speicher vorgesehene und von einem externen Programmiergerät gesendete Parameter empfangen zu können.

Gemäß einer weiteren Ausführungsvariante umfasst das Stimulationssystem ein externes Programmiergerät, welches mit wenigstens einer Eingabeschnittstelle und einer drahtlosen Datenübertragungseinheit ausgestattet ist. Die Datenübertragungseinheit des Programmiergerätes ist dabei auf die entsprechende drahtlose Übertragungseinheit des Stimulationsgerätes abgestimmt. Vorzugsweise ist das externe Programmiergerät nicht nur dazu ausgebildet, Daten an das Stimulationsgerät zu übertragen, sondern die Datenübertragungseinheiten des Programmiergerätes und des Stimulationsgerätes sind für eine bidirektionale Datenübertragung ausgebildet. In diesem Fall verfügt das externe Programmiergerät vorzugsweise auch über eine Parameteranalyseeinheit, welche dazu ausgebildet ist, die vom Stimulationsgerät übertragenen Daten analysieren zu können.

Diese und weitere Aspekte der vorliegenden Erfindung sollen nun anhand der nachfolgenden Figurenbeschreibung näher erläutert werden. Es zeigen:
Figur 1 eine beispielhafte Anordnung von Stimulationsgerät, Sensor und Stimulationselektrode im menschlichen Körper;
Figur 2 das Blockschaltbild einer Ausführungsvariante des erfindungsgemäßen Stimulationsgerätes.

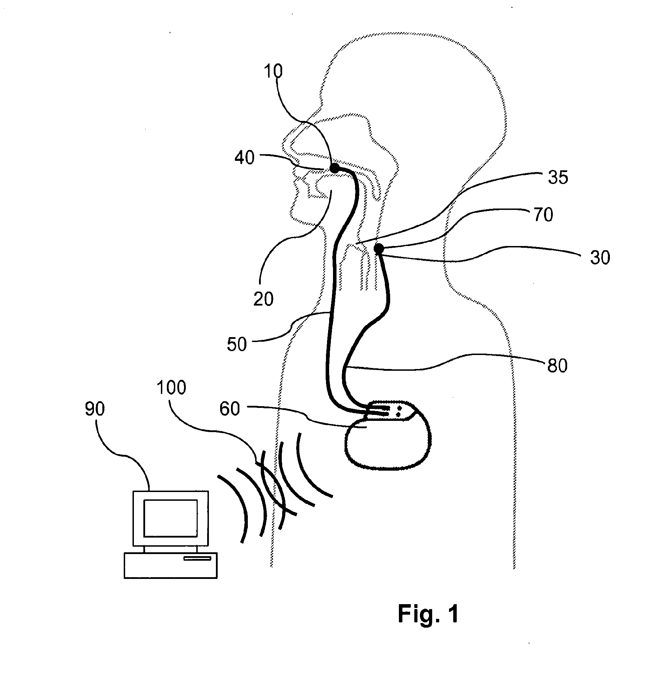

Figur 1 zeigt eine mögliche Anordnung des erfindungsgemäßen Stimulationssystems im Körper eines Patienten. Das Stimulationssystem nach Figur 1 verfügt über ein Stimulationsgerät 60, das über eine Sensorelektrodenleitung 50 mit einem Sensor 40 elektrisch leitend verbunden ist und diesen mit Energie versorgt. Des Weiteren ist das Stimulationsgerät 60 über eine Stimulationselektrodenleitung 80 mit einer Stimulationselektrode 70 verbunden.

Der Sensor 40 ist im einfachsten Fall ein Schalter, welcher nur wenn betätigt ein Sensor-Signal an das Stimulationsgerät 60 weiterleitet. In einem solchen Fall ist jedes bei dem Stimulationsgerät 60 eingehende Sensor-Signal ein auslösendes Sensor-Signal bzw. ein Triggersignal, da es immer auf eine willentlich gesteuerte Muskelbewegung, einen willentlich hervorgerufenen Gehirnstrom oder einen willentlich erzeugten Nervenimpuls zur Initiierung eines Schluckvorgangs zurückgeht. Gemäß der hier dargestellten Ausführungsvariante handelt es sich jedoch um einen Drucksensor, dessen Sensor-Signal kontinuierlich oder zeitlich getaktet über die Sensorelektrodenleitung 50 an das Stimulationsgerät 60 weitergeleitet wird und in diesem aufbereitet wird.

Dabei wertet das Stimulationsgerät 60 die Signale des Sensors 40 aus und erkennt daraus den Druck der Zunge 20 an den Gaumen 10, der für den Beginn eines Schluckvorgangs repräsentativ ist.

Daraufhin erzeugt das Stimulationsgerät 60 einen Stimulationsimpuls und gibt diesen über die Stimulationselektrodenleitung 80 an die Stimulationselektrode 70 aus. In diesem Fall ist die Stimulationselektrode 70 so im Körper eines Patienten implantiert, dass sie den Nervus lanyngeus superior 30 stimuliert.

Der Nervus laryngeus superior 30 wird vom Stimulationsgerät 60 durch die Stimulationselektrode 70 stimuliert und aktiviert dadurch den Schließmuskel 35.

Der Beginn der Stimulation des Nervus laryngeus superior 30 erfolgt dabei nach einer einstellbaren Zeitverzögerung (z. B. 100 ms) gegenüber dem Eingang des auslösenden Sensor-Signals.

Neben dem implantierbaren Stimulationssystem zeigt Figur 1 zusätzlich noch als weiteren Aspekt der vorliegenden Erfindung ein externes Programmiergerät 90.

Das Programmiergerät 90 ist dazu ausgebildet, über eine drahtlose Kommunikationsstrecke 100 mit dem Stimulationsgerät 60 unidirektional und/oder bidirektional zu kommunizieren.

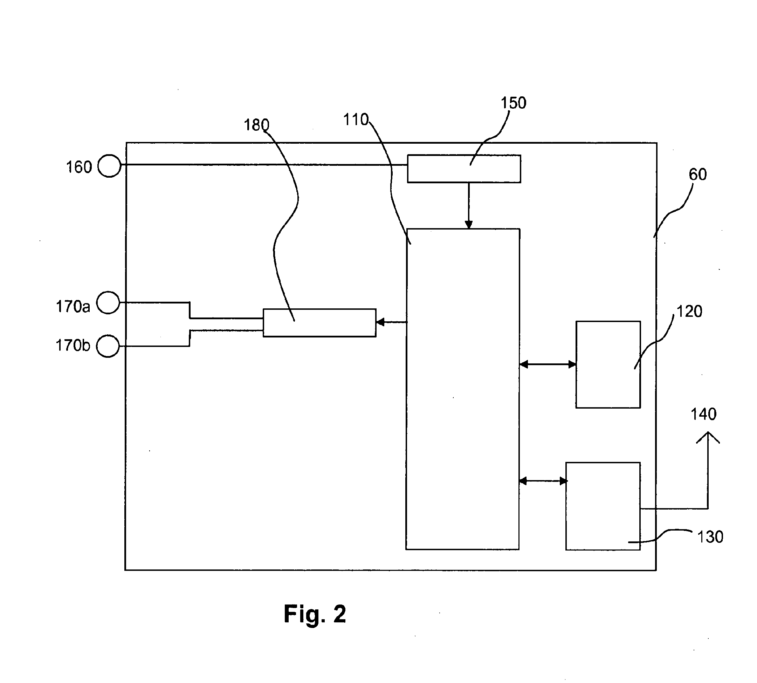

Die Kommunikation 100 zwischen dem Stimulationsgerät 60 und dem Programmiergerät 90 erfolgt über eine in Figur 2 dargestellte drahtlose Datenübertragungseinheit 130, die im Bedarfsfall Signale und/oder Daten vom Programmiergerät 90 über eine Antenne 140 empfängt und an dieses sendet.

Auf diese Weise können Parameter der Stimulation über das Programmiergerät 90 in dem Stimulationsgerät 60 eingestellt werden. Stimulationsparameter können sein: Zeitverzögerung zwischen dem auslösenden Sensor-Signal und der Abgabe des Stimulationsimpulses, Stimulationsfrequenz, Stimulationsart (Strom- oder Spannungsgesteuert), Intensität der Stimulationsimpulse, Dauer eines Stimulationszyklus, Frequenz der Stimulationszyklen.

In einer weiteren Ausführungsform kann das Stimulationsgerät 60 diese Parameter auch selbständig einstellen.

Figur 2 zeigt das Blockschaltbild einer Ausführungsvariante des Stimulationsgerätes 60.

Das Stimulationsgerät 60 enthält eine Recheneinheit 110, die dazu ausgebildet ist, Daten und/oder Parameter in einem Speicher 120 ablegen und wieder aufrufen zu können. Bei den Parametern handelt es sich um Werte, die einen zu erzeugenden Stimulationsimpuls genauer definieren.

Werden im Behandlungsfall die vom Sensor 40 aufgenommen Sensor-Signale über die Elektrodenleitung 50 zum Stimulationsgerät 60 geleitet, so werden sie über den Stecker 160 an die Signalaufbereitung 150 weitergeleitet, aufbereitet und von dort an die Recheneinheit 110 weitergeleitet.

In der Signalaufbereitung 150 wird mit Hilfe verschiedener Schwellenwerte das aufgenommene Sensor-Signal aufbereitet und hinsichtlich des Vorliegens einer willentlich gesteuerten Muskelbewegung, eines willentlich hervorgerufenen Gehirnstroms oder eines willentlich erzeugten Nervenimpulses in der Initialphase eines Schluckvorganges überprüft. Das bearbeitete Sensor-Signal wird daraufhin an die Recheneinheit 110 weitergegeben.

Bei Vorliegen einer initialen Phase eines Schluckvorgangs verwendet die Recheneinheit 110 die in dem Speicher 120 abgelegten Stimulationsparameter und/oder berechnet neue geeignete Signalparameter und gibt diese an einen Stimulationsimpulsformer 180 weiter.

Der Stimulationsimpulsformer 180 formt gemäß den Parametern geeignete Stimulationspulse.

Die Stimulationsimpulse werden daraufhin bei unipolarer Stimulation über einen Stecker 170a oder 170b über die Stimulationselektrodenleitung 80 an die Stimulationselektrode 70 und weiter an den Stimulationsort 30 geleitet.

Bei bipolarer Stimulation wird der Impuls über die beiden Stecker 170a und 170b und über die Stimulationselektrodenleitung 80 an die Stimulationselektrode 70 und weiter an den. Stimulationsort 30 geleitet.

Erfolgt die Behandlung unipolar, befindet sich am Ende der Elektrodenleitung 80 ein Pol 70 von dem aus im Behandlungsfall ein Stimulationsimpuls zum Gehäuse des Implantats abgegeben wird.

Erfolgt die Stimulation bipolar, befinden sich am Ende der Elektrodenleitung 80 (in Figur 1 nicht erkennbar) zwei Pole 70, zwischen denen der Stimulationsimpuls abgegeben wird.

Die Stimulationsimpulse sind vorzugsweise stromgesteuert, das heißt die Stromstärke der Stimulationsimpulse ist einstellbar.

In einer weiteren Ausführungsform sind die Stimulationsimpulse spannungsgesteuert, das heißt die Spannungshöhe der Stimulationsimpulse ist einstellbar.

## Patentansprüche

1. Stimulationssystem zur Behandlung von Dysphagien, mit einem implantierbaren Stimulationsgerät (60), wenigstens einer implantierbaren Stimulationselektrode (70) und wenigstens einem Initialisierungs-Sensor (40), von denen
- der Initialisierungs-Sensor (40) dazu ausgebildet ist, eine willentlich gesteuerte Muskelbewegung, einen willentlich hervorgerufenen Gehirnstrom oder einen willentlich erzeugten Nervenimpuls zur Initiierung eines Schluckvorgangs zu detektieren, kontinuierlich, zeitlich getaktet oder als Reaktion hierauf ein Sensor-Signal zu erzeugen und dieses an das Stimulationsgerät (60) weiterzugeben, wobei
- das Stimulationsgerät (60) dazu ausgebildet ist, auf solch ein Sensor-Signal hin, das eine willentliche Initiierung eines Schluckvorgangs repräsentiert, die Abgabe eines Stimulationsimpulses über die Stimulationselektrode (70) auszulösen,
**dadurch gekennzeichnet, dass** der Initialisierungs-Sensor (40) dazu ausgebildet ist, einen Druck der Zunge gegen den Gaumen zu detektieren.

2. Stimulationssystem nach einem der vorangehenden Ansprüche, **dadurch gekennzeichnet, dass** das Stimulationsgerät (60) einen Zeitgeber umfasst und ausgebildet ist, die Abgabe eines Stimulationsimpulses nach einer vorgegebenen Verzögerungszeit nach Empfang eines auslösenden Sensor-Signals zu bewirken.

3. Stimulationssystem nach einem der vorangehenden Ansprüche, **dadurch gekennzeichnet, dass** das Stimulationsgerät (60) über eine Sensorelektrodenleitung (50) elektrisch leitend mit dem Initialisierungs-Sensor (40) verbunden ist.

4. Stimulationssystem nach einem der Ansprüche 1 bis 2, **dadurch gekennzeichnet, dass** der Initialisierungs-Sensor (40) über eine eigene Energieversorgung verfügt und dazu ausgebildet ist, ein Sensor-Signal drahtlos an das Stimulationsgerät (60) übertragen zu können.

5. Stimulationssystem nach einem der Ansprüche 1 bis 4, **dadurch gekennzeichnet, dass** der Initialisierungs-Sensor (40) ein Drucksensor ist, dessen Sensor-Signal im Initialisierungs-Sensor (40) selbst aufbereitet wird.

6. Stimulationssystem nach einem der vorangehenden Ansprüche, **dadurch gekennzeichnet, dass** der Initialisierungs-Sensor (40) ein Drucksensor ist, welcher dazu ausgebildet ist, kontinuierlich oder zeitlich getaktet ein Sensor-Signal an das Stimulationsgerät (60) abzugeben, wobei das Stimulationsgerät (60) seinerseits dazu ausgebildet ist, das Sensor-Signal des Initialisierungs-Sensors aufzubereiten.

7. Stimulationssystem nach einem der vorangehenden Ansprüche, **dadurch gekennzeichnet, dass** die Stimulationselektrode (70) über eine Stimulationselektrodenleitung (80) elektrisch leitend mit dem Stimulationsgerät (60) verbunden ist.

8. Stimulationssystem nach einem der Ansprüche 1 bis 6, **dadurch gekennzeichnet, dass** die Stimulationselektrode (70) über eine eigene Stromversorgung verfügt und drahtlos mit dem Stimulationsgerät (60) verbunden ist.

9. Stimulationssystem nach einem der vorangehenden Ansprüche, **dadurch gekennzeichnet, dass** das Stimulationssystem (60) über wenigstens zwei Stimulationselek-troden (70) und/oder wenigstens zwei Initialisierungs-Sensoren (40) verfügt.

10. Stimulationssystem nach einem der vorangehenden Ansprüche, **dadurch gekennzeichnet, dass** das Stimulationsgerät (60) über einen Stimulationsimpulsformer (180), eine Recheneinheit (110) und einen Speicher (120) verfügt, wobei der Speicher (120) zum Speichern von Parametern ausgebildet ist, mit Hilfe derer die Recheneinheit (110) im Bedarfsfall die Zeitverzögerung zwischen einem auslösenden Sensor-Signal und der Abgabe des Stimulationsimpulses und/oder die Stimulationsfrequenz und/oder die Stimulationsart und/oder die Dauer eines Stimulationszyklus und/oder die Frequenz der Stimulationszyklen bestimmt und eine entsprechende Impulsgenerierung von dem Stimulationsimpulsformer (180) bewirkt.

11. Stimulationssystem nach einem der vorangehenden Ansprüche, **dadurch gekennzeichnet, dass** das Stimulationsgerät (60) über eine drahtlose Datenübertragungseinheit (130) verfügt, welche dazu ausgebildet ist, die zum Abspeichern in einem Speicher (120) bestimmten, von einem externen Programmiergerät (90) drahtlos gesendete Parameter zu empfangen.

12. Stimulationssystem nach einem der vorangehenden Ansprüche, **dadurch gekennzeichnet, dass** das Stimulationsgerät (60) dazu ausgebildet ist eine selbständige Anpassung der Stimulationsparameter zu bewirken.

13. Stimulationssystem nach Anspruch 11 oder 12 weiters umfassend ein externes Programmiergerät (90) mit wenigstens einer Eingabeschnittstelle und einer drahtlosen Datenübertragungseinheit, welches dazu ausgebildet ist, im Bedarfsfall Stimulations-Parameter drahtlos an das Stimulationsgerät (60) zu übertragen.

14. Stimulationssystem nach Anspruch 13 mit einer drahtlosen Datenempfangseinheit und einer Parameteranalyseeinheit, welches dazu ausgebildet ist, die von dem Stimulationsgerät (60) drahtlos übertragenen Stimulationsparameter empfangen und analysieren zu können.

## Claims

1. A stimulation system for treating dysphagia, having an implantable stimulation device (60), at least one implantable stimulation electrode (70) and at least one initialization sensor (40), of which
- the initialization sensor (40) is designed to detect a deliberately controlled muscle movement, a deliberately generated cerebral flow, or a deliberately generated nerve impulse for the initiation of a swallow process, to generate a sensor signal continuously, in a clock-pulse controlled manner, or in reaction thereto, and to forward it to the stimulation device (60), wherein
- the stimulation device (60) is designed to trigger, in response to such a sensor signal which represents a deliberate initiation of a swallow process, the release of a stimulation pulse via the stimulation electrode (70),
**characterized in that** the initialization sensor (40) is designed to detect pressure of the tongue against the gums.

2. The stimulation system according to one of the preceding claims, **characterized in that** the stimulation device (60) includes a timer and is designed to induce the release of a stimulation pulse after a specified delay time after receipt of a triggering sensor signal.

3. The stimulation system according to one of the preceding claims, **characterized in that** the stimulation device (60) is connected in an electrically conductive manner via a sensor electrode lead (50) to the initialization sensor (40).

4. The stimulation system according to one of the claims 1 to 2, **characterized in that** the initialization sensor (40) has a separate energy supply and is designed to be capable of wirelessly transmitting a sensor signal to the stimulation device (60).

5. The stimulation system according to one of the claims 1 to 4, **characterized in that** the initialization sensor (40) is a pressure sensor, the sensor signal of which is prepared in the initialization sensor (40) itself.

6. The stimulation system according to one of the preceding claims, **characterized in that** the initialization sensor (40) is a pressure sensor which is designed to release a sensor signal to the stimulation device (60) continuously or in a clock-pulse controlled manner, wherein the stimulation device (60) is designed to prepare the sensor signal of the initialization sensor.

7. The stimulation system according to one of the preceding claims, **characterized in that** the stimulation electrode (70) is connected in an electrically conductive manner via a stimulation electrode lead (80) to the stimulation device (60).

8. The stimulation system according to one of the claims 1 to 6, **characterized in that** the stimulation electrode (70) has a separate power supply and is wirelessly connected to the stimulation device (60).

9. The stimulation system according to one of the preceding claims, **characterized in that** the stimulation system (60) has at least two stimulation electrodes (70) and/or at least two initialization sensors (40).

10. The stimulation system according to one of the preceding claims, **characterized in that** the stimulation device (60) has a stimulation pulse shaper (180), an arithmetic unit (110) and a memory (120), wherein the memory (120) is designed to store parameters, with the aid of which the arithmetic unit (110) determines, as needed, the time delay between a triggering sensor signal and the release of the stimulation pulse and/or the stimulation frequency and/or the stimulation type and/or the duration of a stimulation cycle and/or the frequency of the stimulation cycles, and induces a corresponding pulse generation by the stimulation pulse shaper (180).

11. The stimulation system according to one of the preceding claims, **characterized in that** the stimulation device (60) has a wireless data transmission unit (130) which is designed to receive the parameters that are intended for storage in a memory (120) and are transmitted wirelessly by an external programming device (90).

12. The stimulation system according to one of the preceding claims, **characterized in that** the stimulation device (60) is designed to induce an automatic adaptation of the stimulation parameters.

13. The stimulation system according to claim 11 or 12, further comprising an external programming device (90) having at least one input interface and a wireless data transmission unit which is designed to wirelessly transmit stimulation parameters to the stimulation device (60) as needed.

14. The stimulation system according to claim 13 having a wireless data reception unit and a parameter analysis unit which is designed to be capable of receiving and analyzing the stimulation parameters transmitted wirelessly by the stimulation device (60).

## Revendications

1. Système de stimulation pour le traitement de dysphagies, avec un appareil de stimulation implantable (60), au moins une électrode de stimulation implantable (70) et au moins un détecteur d'initialisation (40), parmi lesquels
- le détecteur d'initialisation (40) est conçu pour détecter un mouvement musculaire commandé intentionnellement, un flux cérébral généré intentionnellement ou une impulsion nerveuse produite intentionnellement, pour l'initialisation d'une déglutition, pour produire un signal de détection en continu, de façon cadencée ou réaction à celle-ci, pour transmettre celui-ci à l'appareil de stimulation (60), où
- l'appareil de stimulation (60) est conçu pour déclencher une impulsion de stimulation par l'électrode de stimulation (70) lors d'un tel signal de détection représentant l'initialisation intentionnelle d'une déglutition,
**caractérisé en ce que** le détecteur d'initialisation (40) est conçu pour détecter une pression de la langue contre le palais.

2. Système de stimulation selon l'une des revendications précédentes, **caractérisé en ce que** l'appareil de stimulation (60) comprend un générateur de cadence et est conçu pour provoquer la transmission d'une impulsion de stimulation après un temps de retard prédéfini suite à la réception d'un signal de détection déclencheur.

3. Système de stimulation selon l'une des revendications précédentes, **caractérisé en ce que** l'appareil de stimulation (60) est relié au détecteur d'initialisation (40) de façon électriquement conductrice par une ligne d'électrode de détection (50).

4. Système de stimulation selon l'une des revendications 1 à 2, **caractérisé en ce que** le détecteur d'initialisation (40) dispose d'une alimentation en énergie individuelle et est conçu pour pouvoir transmettre sans fil un signal de détection à l'appareil de stimulation (60).

5. Système de stimulation selon l'une des revendications 1 à 4, **caractérisé en ce que** le détecteur d'initialisation (40) est un détecteur de pression, dont le signal de détection est généré dans le détecteur d'initialisation (40) lui-même.

6. Système de stimulation selon l'une des revendications précédentes, **caractérisé en ce que** le détecteur d'initialisation (40) est un détecteur de pression conçu pour transmettre en continu ou de façon cadencée un signal de détection à l'appareil de stimulation (60), l'appareil de stimulation (60) étant quant à lui conçu pour générer le signal de détection du détecteur d'initialisation.

7. Système de stimulation selon l'une des revendications précédentes, **caractérisé en ce que** l'électrode de stimulation (70) est reliée de façon électriquement conductrice à l'appareil de stimulation (60) par une ligne d'électrode de stimulation (80).

8. Système de stimulation selon l'une des revendications précédentes, **caractérisé en ce que** l'électrode de stimulation (70) dispose d'une alimentation en énergie individuelle et est reliée sans fil à l'appareil de stimulation (60).

9. Système de stimulation selon l'une des revendications précédentes, **caractérisé en ce que** le système de stimulation (60) dispose d'au moins deux électrodes de stimulation (70) et/ou d'au moins deux détecteurs d'initialisation (40).

10. Système de stimulation selon l'une des revendications précédentes, **caractérisé en ce que** l'appareil de stimulation (60) dispose d'un générateur d'impulsions de stimulation (180), d'une unité de calcul (110) et d'une mémoire (120), dans lequel la mémoire (120) est conçue pour l'enregistrement de paramètres, à l'aide desquels l'unité de calcul (110) détermine si nécessaire le retard entre un signal de détection déclencheur et la transmission de l'impulsion de stimulation, et/ou la fréquence de stimulation et/ou le type de stimulation et/ou la durée d'un cycle de stimulation et/ou la fréquence des cycles de stimulation, et entraîne une génération d'impulsion correspondante du générateur d'impulsions de stimulation (180).

11. Système de stimulation selon l'une des revendications précédentes, **caractérisé en ce que** l'appareil de stimulation (60) dispose d'une unité de transmission de données sans fil (130) conçue pour recevoir des paramètres déterminés, envoyés sans fil par un appareil de programmation externe (90) en vue de l'enregistrement dans une mémoire (120).

12. Système de stimulation selon l'une des revendications précédentes, **caractérisé en ce que** l'appareil de stimulation (60) est conçu pour entraîner une adaptation autonome des paramètres de stimulation.

13. Système de stimulation selon la revendication 11 ou 12, comprenant en outre un appareil de programmation externe (90) avec au moins une interface d'entrée et une unité de transmission de données sans fil, conçu pour transmettre si nécessaire des paramètres de stimulation sans fil à l'appareil de stimulation (60).

14. Système de stimulation selon la revendication 13, avec une unité de réception de données sans fil et une unité d'analyse de paramètres, conçu pour pouvoir recevoir et analyser les paramètres de stimulation transmis sans fil par l'appareil de stimulation (60).
